**Europäisches Patentamt**

**European Patent Office**

**Office européen des brevets**

(19)

(11) Numéro de publication: **0 196 965**

**B1**

(12)

# FASCICULE DE BREVET EUROPÉEN

(45) Date de publication du fascicule du brevet:
**12.10.88**

(51) Int. Cl.⁴: **B 01 J 29/18,** C 07 C 5/27,
C 10 G 45/64

(21) Numéro de dépôt: **86400620.0**

(22) Date de dépôt: **24.03.86**

(54) **Nouveau catalyseur d'isomérisation de coupes riches en paraffines normales.**

(30) Priorité: **05.04.85 FR 8505351**

(43) Date de publication de la demande:
**08.10.86 Bulletin 86/41**

(45) Mention de la délivrance du brevet:
**12.10.88 Bulletin 88/41**

(84) Etats contractants désignés:
**BE CH DE GB IT LI LU NL SE**

(56) Documents cité:
**DE-A-1 948 776**
**DE-A-2 745 983**
**FR-A-2 248 314**
**FR-A-2 471 359**

(73) Titulaire: **INSTITUT FRANCAIS DU PETROLE, 4,
Avenue de Bois- Préau, F-92502 Rueil- Malmaison
(FR)**

(72) Inventeur: **Travers, Christine, 12 Bd du Général De
Gaulle, F-92500 Rueil Malmaison (FR)**
Inventeur: **Dufresne, Pierre, 67, rue Georges Sand,
F-92500 Rueil Malmaison (FR)**
Inventeur: **Raatz, Francis, 17, rue Michelet, F-92500
Rueil Malmaison (FR)**
Inventeur: **Marcilly, Christian, 91 ter rue
Condorcet, F-78800 Houilles (FR)**

EP 0 196 965 B1

LIBER, STOCKHOLM 1988

# 0 196 965

## Description

La présente invention concerne un nouveau catalyseur notamment utilisable pour l'hydroisomérisation d'une coupe riche en n-paraffines ayant 4, 5, 6 ou 7 atomes de carbone par molécule. Le catalyseur renferme au moins une zéolithe préparée à partir d'une mordénite petits pores.

En isomérisation, il convient d'ajouter à la zéolithe une matrice et au moins un métal appartenant au groupe VIII de la classification périodique des éléments, plus particulièrement le platine, le palladium et le nickel.

## Description de l'art antérieur

L'isomérisation des paraffines normales de faible poids moléculaire est d'une importance considérable dans l'industrie pétrolière, vu l'indice d'octane particulièrement élevé des isoparaffines.

Il est intéressant de pouvoir transformer les n-paraffines $C_4$-$C_7$ et surtout $C_5$-$C_6$ en isoparaffines afin d'obtenir un carburant à haut indice d'octane. Ce procédé est intéressant pour améliorer les fractions d'essence légère et en particulier, les fractions de tête de distillation directe.

Il existe 3 types différents de procédés d'isomérisation:
- les procédés basse température (environ 20 - 130°C), utilisant un catalyseur de type Friedel et Crafts, tel que le chlorure d'aluminium,
- les procédés "moyenne" température (environ 150°C) utilisant comme catalyseur un métal supporté tel que le platine sur alumine halogénée,
- les procédés haute température (250°C et plus) mettant en oeuvre des supports zéolithiques associés à un métal hydrogénant du groupe VIII.

L'équilibre thermodynamique est plus favorable aux isoparaffines à basse température, cependant les catalyseurs de type Friedel et Crafts à support alumine halogénée, de par leur nature corrosive, nécessitent des réacteurs en alliages spéciaux très coûteux.

Les procédés "haute température" font depuis une vingtaine d'années l'objet de nombreux brevets. La plupart des catalyseurs décrits sont constitués de zéolithe, plus particulièrement de mordénite, sous forme acide, avec ou sans promoteurs d'hydrogénation. SHELL s'est particulièrement intéressé à ce procédé et a revendiqué des catalyseurs à base de mordénite, et plus particulièrement:
- le mode d'échange des ions $Na^+$ par les ions $NH_4^+$ ou $H^+$ (US-3 190 939),
- le traitement acide:    succession acide chaud, $NH_4^+$ (US-3 442 794),
      : succession acide chaud-acide froid (US-375 345),
      : solution d'acide contenant des ions $Na^+$ ou $K^+$ (US-2 772 737; US-4 359 409; US-4 400 576)

- les traitements thermiques à humidité contrôlée (US-3 836 597; US-3 842 114; US-2 181 928).

ESSO, pour sa part, à revendique les procédés de désalumination de la mordénite suivants:
- traitements acides sévères: HCl 12N, 100°C (US-3 480 539),
- self steaming (calcination en atmosphère confinée) entre 430°C et 820°C suivi d'une attaque acide (US-3 506 400).

On trouve également dans le brevet US-3 551 353 de MOBIL, la succession self steaming-attaque acide pour désaluminer la zéolithe.

Deux modes de dépôt de métal sont envisagés:
- dépôt du métal sur la zéolithe modifiée, c'est le cas de la plupart des brevets précités,
- dépôt du ou des métaux sur un liant inerte, alumine par exemple et mélange physique avec la zéolithe sous forme protonique (US-3 432 568 MOBIL, US-3 632 835 UOP, US-4 374 926 MOBIL).

Des traitements particuliers de la zéolithe, tels que l'introduction de fluor ont également été brevetés (US-3 932 554 Nippon oil, US-3 413 370 UOP).

La mordénite est caractérisée par un rapport atomique Si/Al compris généralement entre 4 et 6; sa structure cristalline est constituée d'enchaînements de tétraèdres de base $SiO_4$ et $AlO_4$, générateurs de deux types de canaux des canaux à ouverture dodécagonale (contour à 12 oxygènes) et des canaux à ouverture octogonale (contour à 8 oxygènes).

Il existe cependant 2 types de mordénite, qui se distinguent par leurs propriétés d'adsorption la forme à larges pores, toujours synthétique) qui adsorbe les mol écul es telles que le benzène (diamètre cinétique = 6,6 x $10^{-10}$ m et la forme à petits pores, naturelle ou synthétique, qui n'adsorbe que des molécules de diamètre cinétique inférieur à 4,4 x $10^{-10}$ m environ. Ces mordénites se distinguent également par des différences morphologiques - aiguilles pour la mordénite petits pores, sphérulites pour la mordénite larges pores - et structurales présence ou non de défauts. Dans toute la littérature précitée, c'est la mordénite larges pores qui est utilisée. Or l'objet de notre invention réside dans le fait d'utiliser une mordénite larges pores préparée à partir d'une mordénite petits pores et préparée dans des conditions telles que la mordénite larges pores utilisée aura conservé la morphologie de la mordénite à petits pores tout en possédant la capacité d'absorber la molécule de benzène (diamètre cinétique: 6,6 x $10^{-10}$ m) ce qui n'est pas le cas d'une mordénite petits pores

2

comme indiqué ci-dessus. L'utilisation de cette mordénite de morphologie particulière (aiguilles), spécialement traitée, entraîne un gain d'activité et de sélectivité important pour la réaction d'isomérisation.

Il est possible de "déboucher" les canaux de cette zéolithe particulière par traitement dans un acide minéral fort et/ou par calcination en présence de vapeur d'eau et d'accéder à une capacité d'adsorption voisine de celle de la mordénite larges pores.

Ces mordénites synthétiques à petits pores peuvent être obtenues par synthèse notamment dans les conditions suivantes température comprise entre 200 et 300°C environ, et temps de cristallisation de 5 à 50 heures.

La zéolithe utilisée dans le catalyseur de la présente invention est fabriquée à partir d'une mordénite petits pores dont la teneur en sodium est comprise généralement entre 4 et 6,5 pour cent (poids) par rapport au poids de mordénite sèche, dont le rapport atomique Si/Al est compris généralement entre 4,5 et 6,5 et le volume de maille généralement compris entre 2,80 et 2,77 nm$^3$. Cette mordénite n'adsorbe que des molécules de diamètre cinétique inférieur à environ 4,4 x 10$^{-10}$ m. Après traitements, la mordénite est caractérisée par différentes spécifications dont les méthodes de détermination seront précisées dans la suite du texte un rapport atomique Si/Al compris entre 5 et 50 et de préférence entre 5 et 30, une teneur en sodium inférieure à 0,2 % poids et de manière préférée inférieure à 0,1 % par rapport au poids de zéolithe sèche, un volume de maille, V, de la maille élémentaire compris entre 2,78 et 2,73 nm$^3$ et de manière préférée entre 2,77 et 2,74 nm$^3$, une capacité d'adsorption de benzène supérieure à 5 % et de préférence à 8 % par rapport au poids de solide (zéolithe) sec, une morphologie particulière, à savoir qu'elle se présente en majeure partie sous forme d'aiguilles de préférence de longueur moyenne 5 microns (5 x 10$^{-6}$ m) dont les faces hexagonales ont une longueur d'environ 1 µm (1 x 10$^{-6}$ m) et une "hauteur" d'environ 0,3 µm (0,3 x 10$^{-6}$ m).

Les différentes caractéristiques des zéolithes sont mesurées par les méthodes ci-après:
- les rapports atomiques Si/Al globaux sont déterminés par analyse fluorescence X, les teneurs en sodium par absorption atomique,
- le volume de la maille et la cristallinite sont déterminés par diffraction X, l'échantillon étant préparé de manière analogue au mode opératoire de la norme ASTM D 3942 80 établie pour la faujasite,
- la capacité d'adsorption en benzène de la zéolithe est déterminée par gravimètrie. L'échantillon est préalablement désorbé à 300°C sous 10$^{-4}$ Torr (133,32 x 10$^{-4}$ Pa).

L'adsorption est ensuit menée à 30°C pendant 4 heures sous une pression P de 28 Torr (V3733 Pa) de benzène, ce qui correspond à un rapport P/Ps égal à 0,25, Ps étant la pression de vapeur saturante à la température de l'expérience. Les volumes adsorbés sont calculés à partir de la densité de l'adsorbat sous forme liquide à la température de l'adsorption: d = 0,868.

La mordénite ainsi préparée et destinée à être utilisée dans les réactions d'hydroisomérisation, est ensuite mélangée à une matrice généralement amorphe, le catalyseur d'hydroisomérisation renfermant également de préférence au moins un métal du groupe VIII, et de préférence du platine, du palladium ou du nickel. Dans le cas du platine et du palladium, la teneur (en poids) est comprise entre 0,05 et 1 % et de manière préférée entre 0,1 et 0,6 %. Dans le cas du nickel la teneur pondérale est comprise entre 0,10 et 10 % et de manière préférée entre 0,2 et 5 %. Ce catalyseur constitue un nouveau catalyseur d'hydroisomérisation de coupes riches en paraffines normales à 4 ou 7 atomes de carbone et de préférence à 5 ou 6 atomes de carbone par molécule, présentant une sélectivité et une activité exaltées par rapport aux catalyseurs d'hydroisomérisation classiques.

Il existe diverses méthodes d'obtention d'une zéolithe larges pores telle que définie ci-dessus à partir d'une mordénite petits pores. Selon une méthode préférée la mordénite petits pores utilisée, est soumise aux différents traitements suivants les cations sodium sont échangés par des cations ammonium, en plongeant la zéolithe dans une solution d'un sel d'ammonium ionisable de molarité généralement supérieure à 0,5, à une température généralement comprise entre 20°C et 150°C. Cet échange peut être répété plusieurs fois. Le produit ainsi obtenu après ces échanges cationiques, peut être lavé puis soumis à un traitement thermique en présence de vapeur d'eau, qui peut être effectué par la technique du self steaming (calcination en atmosphère confinée). La température est comprise entre 300 et 800°C et de préférence 400 et 700, pendant un temps supérieur généralement à 10 minutes et de préférence supérieur à 20 minutes. L'atmosphère de calcination contient au moins 1 % et de préférence au moins 5 % de vapeur d'eau. Dans le cas du self steaming, l'atmosphère est constituée essentiellement d'eau et d'ammoniac. Le produit ainsi obtenu peut être soumis à un traitement acide visant à extraire de l'aluminium du solide. Ce traitement peut s'effectuer en plongeant le produit dans un acide minéral ou organique fort de normalité comprise entre 0,1 et 12 N, à une température comprise entre 20 et 150°C et de préférence entre 80 et 150°C, pendant un temps supérieur de préférence à 10 minutes.

Ce produit ayant subi le traitement acide peut être lavé, par exemple à l'acide, puis lavé à l'eau, puis éventuellement mélangé à toute matrice adéquate. Il est ensuite mis en forme et éventuellement chargé par exemple de platine et/ou de palladium et/ou de nickel, comme on le verra plus loin soit, avant soit après l'introduction de la matrice, soit sur la matrice elle-même. Selon une autre méthode de préparation de la zéolithe selon l'invention, il est possible également d'obtenir un bon catalyseur en utilisant un protocole différent. La mordénite petits pores forme sodique peut être traitée directement une ou plusieurs fois par un acide minéral ou organique, de normalité comprise entre 0,1 et 12 N à une température comprise entre 20 et 150°C et de préférence 80 et 150°C. Dans ce cas, la quantité d'aluminium extraite par ce traitement acide doit être au moins de 20 %, ce qui veut dire que le rapport Si/Al doit être d'au moins environ 6,5. Il est éventuellement possible de parfaire ensuite l'échange des cations sodium en traitant le produit dans des solutions d'un sel d'ammonium ionisable. L'introduction d'un métal se déroule ensuite selon un des protocoles décrits plus haut.

D'autres méthodes de désalumination peuvent être envisagées telles que l'attaque par l'acide fluorhydrique, l'acide chlorhydrique en phase gazeuse, ou le traitement par un fluorosilicate ou toute autre méthode connue de l'homme de l'art.

La zéolithe modifiée, lorsqu'elle est conçue pour être utilisée dans les réactions d'hydroisomérisation est mélangée intimement à une matrice, par exemple à une poudre humide d'un gel d'alumine. Le mélange est ensuite mis en forme par exemple par extrusion au travers d'une filière. La teneur en mordénite du support ainsi obtenu doit être supérieure à 40 % et de préférence supérieure à 60 % (en poids). La mise en forme peut être réalisée avec d'autres matrices que l'alumine telles que la silice-alumine, les argiles naturelles (le kaolin ou la bentonite), l'alumine-oxyde de bore,... et avec une technique autre que l'extrusion telle que le pastillage, la dragéification ou toute autre technique connue de l'homme de l'art.

Le métal hydrogénant est ensuite déposé sur ce support. N'importe quel métal du groupe VIII peut convenir mais en particulier le platine, le palladium et le nickel.

Le platine peut être introduit de différentes façons:
- sous forme de complexe tetrammine par échange cationique le métal se déposera alors préférentiellement sur la mordénite,
- sous forme d'acide hexachloroplatinique: par échange anionique, le métal se déposera alors préférentiellement sur l'alumine si l'alumine est le liant utilisé lors de la mise en forme.

La première méthode peut être appliquée pour déposer le métal soit sur la poudre de zéolithe, soit sur un produit déjà mis en forme, avec ou sans cation compétiteur ammonium. Sur extrudés ou sur poudre, le métal peut également être déposé par la technique dite de l'imprégnation à sec. Le produit séché est ensuite calciné entre 300°C et 600°C.

Suivant l'invention, la charge riche en paraffines légères à 5 ou 6 atomes de carbone et l'hydrogène sont mis en contact avec un catalyseur du type décrit ci-dessus dans les conditions de l'isomérisation. Ce contact peut s'effectuer en utilisant le catalyseur en lit fixe, en lit fluidisé ou en batch (c'est-à-dire en discontinu).

Le procédé est mis en oeuvre entre 200 et 300°C et de préférence entre 230 et 280°C, à des pressions partielles d'$H_2$ comprises entre la pression atmosphérique et 70 bars et de préférence entre 5 et 50 bars. La vitesse spatiale peut être comprise entre 0,5 et 10 litres d'hydrocarbures liquides par litre de catalyseur et par heure et de préférence entre 1 et 5. Le rapport molaire $H_2$/charge peut varier entre de larges limites et est compris normalement entre 0,5 et 10 et de préférence entre 1 et 3. L'isomérisation étant une réaction équilibrée, l'isomérisat contient encore une quantité importante de n-paraffines non converties. Ces paraffines peuvent être séparées des isomères par exemple par distillation ou par fractionnement sur tamis moléculaire et recyclées dans l'unité d'isomérisation.

Les exemples qui suivent définissent l'invention sans en limiter la portée.

Les performances sont exprimées en terme de conversion du n-hexane et de sélectivité en isomérisation et définies comme suit:

$$\text{Conversion} = \frac{\text{Masse de n-hexane entrée-masse de n-hexane sortie} \times 100}{\text{Masse de n-hexane entrée}}$$

$$\text{Sélectivité} = \frac{\Sigma(\text{Masse des isomères}) \times 100}{\Sigma(\text{Masse des produits de la réaction})}$$

**EXEMPLE 1**: Préparation du catalyseur A selon l'invention

La matière première est une mordénite petits pores référence Alite 150 de la Société Chimique de la Grande Paroisse. Sa formule chimique à l'état anhydre est: Na $AlO_2(SiO_2)_{5,5}$, et sa capacité d'adsorption de benzène est de 1 % en poids par rapport au poids de solide sec (volume de maille 2,79 nm$^3$; teneur en sodium 5,3 % (poids), diamètre cinétique des molécules adsorbées: 3,8 x 10$^{-10}$ m); 50 g de cette poudre sont plongés dans une solution 2M de nitrate d'ammonium et la suspension est portée à 95°C pendant deux heures.

Le volume de la solution de nitrate d'ammonium engagée est égal à 4 fois le poids de zéolithe sec (V/P = 4). Cette opération d'échange cationique est recommencée 3 fois. Après le 3ème échange, le produit est lavé à l'eau, à 20°C pendant 20 minutes avec un rapport V/P égal à 4. La teneur en sodium, exprimée en pourcentage en poids par rapport au poids sec passe de 5,5 à 0,1 %. Le produit est ensuite filtré et soumis à une calcination en atmosphère confinée (self steaming) à 600°C pendant 2 heures.

On procède ensuite à une attaque acide avec de l'acide chlorhydrique 0,58N, en portant le produit à reflux dans la solution aqueuse d'acide chlorhydrique à 90°C pendant 2 heures avec un rapport V/P égal à 8. Le produit est ensuite filtré, lavé à l'acide chlorhydrique 0,1N puis à l'eau.

Le rapport atomique Si/Al de cette mordénite est égal à 12, son volume de maille à 2,750 nm$^3$, son taux de sodium à 300 ppm et sa capacité d'adsorption de benzène à 9,6 % poids par rapport au poids de solide sec. La morphologie de cette mordénite est en forme d'aiguilles de longueurs moyennes 5 x 10$^{-6}$ m dont les faces sont hexagonales et ont une longueur d'environ 1 x 10$^{-6}$ m et une hauteur d'environ 0,3 x 10$^{-6}$ m. Cette mordénite ainsi modifiée est ensuite malaxée avec un liant de type bentonite, puis ce mélange, contenant 25 % poids de bentonite est forcé au travers d'une filière. Les extrudés de diamètre 1,2 x 10$^{-3}$ m sont ensuite séchés et calcinés.

0,4 % de platine sont ensuite déposés sur ce support par échange cationique à partir de chlorure de platine tétramine $Pt(NH_3)_4Cl_2$ avec du nitrate d'ammonium comme ion compétiteur. La quantité de sodium dans le catalyseur final est égale à 80 ppm. Le rapport atomique Si/Al est égal à 12 et le volume de maille a 2,750 nm$^3$. Les extrudés sont ensuite séchés puis calcinés à 500°C.

Le catalyseur ainsi obtenu est chargé dans une unité catalytique en lit fixe et réduit sous hydrogène à 450°C. Il est ensuite testé avec une charge de normal hexane dans les conditions suivantes température 250°C, pression 30 bars, poids de n-hexane par unité de poids de mordénite et par heure: 2, rapport molaire hydrogène sur normal hexane: 2. Les performances indiquées dans le tableau 1 sont prises après 30 h de mise en régime du catalyseur.

**EXEMPLE 2**: Préparation du catalyseur B selon l'invention

L'exemple n° 2 diffère de l'exemple n° 1 en ce que la désalumination de la mordénite n'est plus obtenue par succession des traitements, échanges, self-steaming, attaque acide mais uniquement par un traitement acide consistant en 3 attaques successives avec HCl/N à 100°C pendant 2 h. Le rapport atomique Si/Al est de 12, le volume de maille de 2,759 nm$^3$, la capacité d'adsorption de benzène de 7,9 %. Cette mordénite a une morphologie en forme d'aiguilles dont les dimensions sont sensiblement les mêmes que celles du catalyseur A. La suite de la préparation est analogue à celle de l'exemple 1. Le taux de platine est de 0,4 % et le taux de sodium est égal à 90 ppm. Les performances résumées dans le tableau 1 sont légèrement inférieures à celles obtenues avec le catalyseur A.

**EXEMPLE 3**: Préparation du catalyseur C non conforme à l'invention

Dans l'exemple 3, la mordénite petits pores décrite dans l'exemple 1 est juste échangée 4 fois dans des solutions 2M de nitrate d'ammonium, mais non soumise à des traitements acide ou thermique. Le rapport Si/Al est égal à 5,5, le taux de sodium à 300 ppm et le volume de maille à 2,778 nm$^3$, la capacité d'adsorption de benzène à 1,2 % poids. Le catalyseur est ensuite préparé comme dans l'exemple 1, avec un taux de platine égal à 0,4 %. Les performances résumées dans le tableau I, montrent que du fait de la structure bouchée de la mordénite petits pores, l'activité en isomérisation est très faible.

**EXEMPLE 4**: Préparation du catalyseur D conforme à l'invention

Le catalyseur D diffère du catalyseur A décrit dans l'exemple 1, en ce qu'on réalise 5 échanges successifs par des solutions 2M de nitrate d'ammonium à 95°C, suivis d'un self steaming à 600°C. Le rapport atomique Si/Al est alors de 5,5, le volume de maille de 2,755 nm$^3$, la capacité d'adsorption de benzène de 7,20 %. Ce catalyseur présente la morphologie en aiguilles dont les dimensions sont sensiblement les mêmes que celles du catalyseur A. Le catalyseur est ensuite préparé comme dans l'exemple 1. Le catalyseur contient 0,4 % de platine et 90 ppm de sodium. Les performances obtenues sont inférieures à celles obtenues avec le catalyseur A selon l'invention et sont voisines de celles obtenues avec le catalyseur B.

**EXEMPLE 5**: Préparation du catalyseur E, non conforme à l'invention

Le catalyseur E diffère du catalyseur A décrit dans l'exemple 1 en ce que la mordénite utilisée est une mordénite larges pores sous forme de poudre référencée Zeolon 100 Na de la société NORTON.

50 g de cette poudre sont portés à reflux 2 heures à 950°C dans une solution de nitrate d'ammonium. Cet échange est recommencé 3 fois. Après le dernier échange le produit est lavé à l'eau pendant 20 mn à 20°C, filtré et calciné en atmosphère confinée (self steaming) à 600°C pendant 2 heures. Ce traitement thermique est suivi d'une attaque acide avec de l'acide chlorhydrique 0,58 M. Le solide est porté à reflux dans une solution aqueuse d'acide chlorhydrique à 90°C pendant 2 heures, puis lavé à l'eau.

Le rapport atomique Si/Al de la zéolithe obtenue est égal à 12, le volume de la maille à 2,752 nm$^3$, et le taux de sodium à 95 ppm. Ce produit contrairement aux mordénites selon l'invention, n'a pas une morphologie conforme d'aiguilles. Le produit obtenu est ensuite mis en forme par malaxage avec un gel d'alumine préalablement peptisé.

Ce mélange contenant 25 % poids d'alumine est forcé au travers d'une filière. Les extrudés de diamètre 1,2 x 10$^{-3}$ m sont ensuite séchés et calcinés. 0,4 % de platine sont ensuite déposés sur ce produit suivant la technique décrite dans l'exemple 1.

Les performances énoncées à isoconditions dans le tableau I, sont de 20 points inférieures à celles obtenues avec le catalyseur A selon l'invention.

**EXEMPLE 6**: Préparation du catalyseur F, non conforme à l'invention

Le catalyseur F diffère du catalyseur E décrit dans l'exemple 5, uniquement en ce qu'il n'a pas subi d'échanges et de traitements thermiques, mais uniquement trois traitements acides successifs. La zéolithe larges pores en poudre est porté à reflux à 100°C pendant 2 h dans de l'acide chlorhydrique N. Le protocole de dépôt du platine est identique à celui décrit dans les exemples précédents. Le rapport Si/Al atomique de la zéolithe obtenue est égal à 12, le volume de la maille élémentaire à $2,751\ nm^3$, la teneur en sodium de 100 ppm et la teneur en platine de 0,4 %. Cette zéolithe n'a pas une conformation en aiguilles.

Les performances présentées dans le tableau I sont équivalentes à celles obtenues avec le catalyseur E, mais de 20 points inférieures à celles obtenues avec le catalyseur A selon l'invention.

**TABLEAU I**

| Exemple | Catalyseur | Mordénite Matière Première | Traitement subi | | | Conversion | Sélectivité |
|---------|-----------|----------|------|------|------|------|------|
| | | | Echange $NH_4{}^+$ | Calcination en atmosphère confinée | Traitement acide | | |
| 1 | A | Alite 150 | X | X | X | 79,5 | 9,86 |
| 2 | B | Alite 150 | | | X | 70,2 | 97,3 |
| 3 | C | Alite 150 | X | | | 7 | 97 |
| 4 | D | Alite 150 | X | X | | 68 | 97 |
| 5 | E | Zéolon 100 Na | X | X | X | 56,2 | 96,5 |
| 6 | F | Zéolon 100 Na | | | X | 55,5 | 96,5 |

**Revendications**

1. Catalyseur à base d'une mordénite à larges pores, adsorbant des molécules de diamètre cinétique supérieur à environ $6,6 \times 10^{-10}$ m, avant un rapport atomique Si/Al compris entre environ 5 et 50, une teneur en sodium inférieur a 0,2 % en poids, par rapport à la totalité de la mordénite sèche, un volume de maille V, de la maille élémentaire, compris entre 2,78 et $2,73\ nm^3$, une capacité d'adsorption de benzène supérieure à 5 % poids par rapport au poids de mordénite sèche, la mordénite se présentant, en majeure partie, sous forme d'aiguilles.

2. Catalyseur selon la revendication 1 à base d'une mordénite à larges pores, adsorbant des molécules de diamètre cinétique supérieur à environ $6,6 \times 10^{-10}$ m, un rapport atomique Si/Al compris entre 5 et 30, une teneur en sodium inférieure à 0,1 % en poids par rapport à la totalité de mordénite sèche, un volume de la maille élémentaire compris entre 2,77 et $2,74\ nm^3$, une capacité d'adsorption de benzène supérieure à 8 % par rapport au poids de mordénite sèche, la mordénite se présentant en majeure partie sous forme d'aiguilles de longueur moyenne $5 \times 10^{-6}$ m dont les faces, en majeure partie hexagonales, ont une longueur d'environ $1 \times 10^{-6}$ m et une hauteur d'environ $0,3 \times 10^{-6}$ m.

3. Catalyseur contenant (a) une matrice, (b) une mordénite selon l'une des revendications 1 et 2 et (c) au moins un métal du groupe VIII de la classification périodique des éléments.

4. Catalyseur selon la revendication 3 dans lequel le métal est choisi dans le groupe constitué par le platine, le palladium et le nickel.

5. Catalyseur selon la revendication 4 dans lequel la teneur en métal du groupe VIII est comprise, en poids par rapport à la masse totale du catalyseur, entre 0,05 et 1 % dans le cas où ce métal est le platine ou le palladium, entre 0,10 et 10 % dans le cas où le métal est le nickel.

6. Procédé de fabrication d'une mordénite dite "larges pores" selon l'une des revendications 1 à 5 à partir d'une mordénite dite "petits pores" dont la teneur en sodium est comprise généralement entre 4 et 6,5 % en poids par rapport à la mordénite sèche, dont le rapport atomique Si/Al est compris généralement entre 4,4 et 6,5 et le volume de maille est généralement compris entre 2,80 et $2,77\ nm^3$, ladite mordénite adsorbant des molécules de diamètre cinétique inférieur à $4,4 \times 10^{-10}$ m environ, le procédé étant caractérisé en ce que:

(a) on procède au moins une fois à un échange des cations sodium de la mordénite dite "petits pores" par des cations ammonium,

(b) on traite le produit obtenu à l'étape (a) entre 300 et 800°C dans une atmosphère contenant une teneur en eau d'au moins 1 %,

(c) on traite le produit ainsi obtenu par un acide minéral ou organique de normalité comprise entre 0,1 et 12 N, entre 20 et 150°C,

(d) la masse obtenue est mise en forme.

7. Procédé selon la revendication 6 dans lequel, dans l'étape (b), ladite atmosphère contient une teneur en eau d'au moins 5 %.

8. Utilisation du catalyseur selon l'une des revendications 1 à 5 ou préparé selon l'une des revendications 6 et 7, dans les réactions d'hydroisomérisation d'une coupe riche en n-paraffines possédant 4 à 7 atomes de carbone par molécule.

## Patentansprüche

1. Katalysator auf der Basis eines Modenits mit großen Poren, der Moleküle mit einem kinetischen Durchmesser von über etwa $6,6 \times 10^{-10}$ m absorbiert und ein Atomverhältnis Si/Al zwischen etwa 5 und 50, einen Natriumgehalt unterhalb von 0,2 Gew.-%, bezogen auf die Gesamtheit des getrockneten Mordenits, ein Käfigvolumen V des Elementarkäfigs zwischen 2,78 und 2,73 nm³, eine Adsorptionskapazität für Benzol über 5 Gew.-%, bezogen auf das Gewicht des getrockneten Mordenits, aufweist, wobei der Mordenit zum überwiegenden Teil in Form von Nadeln vorliegt.

2. Katalysator nach Anspruch 1 auf der Basis eines Mordenits mit großen Poren, der Moleküle mit einem kinetischen Durchmesser über etwa $6,6 \times 10^{-10}$ m adsorbiert, ein Atomverhältnis Si/Al zwischen 5 und 30, einen Natriumgehalt unterhalb 0,1 Gew.-%, bezogen auf die Gesamtheit des trockenen Mordenits, ein Volumen des Elementarkäfigs zwischen 2,77 und 2,74 nm³ und eine Adsorptionskapazität für Benzol über 8 %, bezogen auf das Gewicht des trockenen Mordenits aufweist, wobei der Mordenit größtenteils in Form von Nadeln von einer mittleren Länge von $5 \times 10^{-6}$ m vorliegt, deren Flächen, die größtenteils hexagonal sind, eine Länge von etwa $1 \times 10^{-6}$ m und eine Höhe von etwa $0,3 \times 10^{-6}$ m haben.

3. Katalysator, der (a) eine Matrix und (b) einen Mordenit nach einem der Ansprüche 1 und 2 und (c) zumindest ein Metall der Gruppe VIII des periodischen Systems der Elemente aufweist.

4. Katalysator nach Anspruch 3, worin das Metall aus der Gruppe Platin, Palladium und Nickel gewählt ist.

5. Katalysator nach Anspruch 4, worin der Gehalt an Metall der Gruppe VIII, in Gew.-% bezogen auf die Gesamtmasse des Katalysators, zwischen 0,05 und 1 % für den Fall, wo das Metall platin oder Palladium ist und zwischen 0,10 und 10 % für den Fall, wo das Metall Nickel ist, liegt.

6. Verfahren zur Herstellung eines "großporig" genannten Mordenits nach einem der Ansprüche 1 bis 5 ausgehend von einem "kleinporig" genannten Mordenit, dessen Gehalt an Natrium im allgemeinen zwischen 4 und 6 Gew.-%, bezogen auf den trockenen Mordenit, dessen Atomverhältnis Si/Al im allgemeinen zwischen 4,4 und 6,5 und dessen Käfigvolumen im allgemeinen zwischen 2,80 und 2,77 nm³ liegen, wobei dieser Mordenit Moleküle mit einem kinetischen Durchmesser unterhalb etwa $4,4 \times 10^{-10}$ m absorbiert, dadurch gekennzeichnet, daß man:

(a) zumindest einmal Natriumkationen des "kleinporig" genannten Mordenits durch Ammoniumionen austauscht,

(b) das in Stufe (a) erhaltene Produkt zwischen 300 und 800°C in einer Atmosphäre behandelt, die einen Wassergehalt von mindestens 1 % aufweist,

(c) das so erhaltene Produkt mit einer Mineralsäure oder organischen Saure einer Normalität zwischen 0,1 und 12 N bei 20 bis 150°C behandelt und

(d) die erhaltene Masse formt.

7. Verfahren nach Anspruch 6, dadurch gekennzeichnet, daß in Stufe (b) die Atmosphäre einen Wassergehalt von mindestens 5 % aufweist.

8. Verwendung des Katalysators nach einem der Ansprüche 1 bis 5 oder hergestellt nach einem der Ansprüche 6 und 7 bei Hydroisomerisationsreaktionen einer Fraktion, die reich an n-Paraffinen mit 4 bis 7 Kohlenstoffatomen pro Molekül ist.

## Claims

1. A catalyst whose basic constituent is a mordenite of the wide pores type, adsorbing molecules of a kinetic diameter larger than about 0.66 nm, having a Si/Al atomic ratio from about 5 to 50, a sodium content lower than 0.2 % by weight with respect to the total amount of the dry mordenite, a mesh volume V of elementary mesh from 2.78 to 2.73 nm³ a benzene adsorption capacity higher than 5 % by weight with respect to the weight of the dry mordenite, the mordenite being in major part of needle configuration.

2. A catalyst according to claim 1, having a Si/Al atomic ratio from 5.5 to 30, a sodium content lower than 0.1 % by weight with respect to the total amount of dry mordenite, a volume of elementary mesh from 2.77 to 2.74 nm³, a benzene adsorption capacity higher than 8 % with respect to the weight of dry mordenite, the mordenite being in major part formed as needles whose average length is 5 μm and whose faces, in major part hexagonal, have a length of about 1 μm and a height of about 0.3 μm.

3. A catalyst containing (a) a matrix, (b) a mordenite according to one of claims 1 and 2 and (c) at least one metal from group VIII of the periodic classification of elements.

4. A catalyst according to claim 3, therein the metal is selected from the group consisting of platinum, palladium and nickel.

7

5. A catalyst according to claim 4, therein the group VIII metal content ranges, in proportion by weight with respect to the total catalyst weight, from 0.05 to 1 % when the metal is platinum or palladium and from 0.10 to 10 % when the metal is nickel.

6. A process for manufacturing a so-called "wide pores" mordenite according to one of claims 1 in 5 from a so-called "small pores" mordenite whose sodium content is generally from 4 to 6.5 % by weight with respect to the dry mordenite, wose Si/Al atomic ratio is generally from 4.4 to 6.5 and whose mesh volume is generally from 2.80 to 2.77 nm$^3$, said mordenite adsorbing molecules of kinetic diameter lower than about 0.44 nm, said process being characterized by the steps of:

(a) exchanging one or more times sodium cations of the so-called "small pores" mordenite with ammonium cations,

(b) treating the product obtained in step (a) at a temperature from 300 to 800°C in an atmosphere whose water content is at least 1 %,

(c) treating the resultant product with an inorganic or organic acid of normality ranging from 0.1 to 12 N, at a temperature from 20 to 150°C, and

(d) shaping the resultant mass.

7. A process according to claim 6, wherein, in step (b), the water content of said atmosphere is at least 5 %.

8. The use of a catalyst according to one of claims 1 to 5, or prepared according to one of claims 6 and 7, for hydroisomerizing a cut containing a large proportion of n-paraffins having 4 to 7 carbon atoms per molecule.